# EUROPÄISCHE PATENTSCHRIFT

(11) **EP 2 601 308 B1**
(45) Veröffentlichungstag und Bekanntmachung des Hinweises auf die Patenterteilung: **25.11.2015**
(21) Anmeldenummer: 11738243.2
(22) Anmeldetag: 02.08.2011
(51) Int. Cl.: C12Q 1/68

(54) **ASPP2-SPLICEVARIANTE**
ASPP2 SPLICE VARIANT
VARIANT D'EPISSAGE DE ASPP2

(30) Priorität: 02.08.2010 DE 102010033575
(43) Veröffentlichungstag der Anmeldung: 12.06.2013
(73) Patentinhaber: Eberhard-Karls-Universität Tübingen Universitätsklinikum, 72076 Tübingen (DE)
(72) Erfinder: KAMPA-SCHITTENHELM, Kerstin, 72072 Tübingen (DE); SCHITTENHELM, Marcus, 72072 Tübingen (DE)
(74) Vertreter: Witte, Weller & Partner Patentanwälte mbB
(86) Internationale Anmeldenummer: PCT/EP2011/063283
(87) Internationale Veröffentlichungsnummer: WO 2012/016979

(56) Entgegenhaltungen:
- WO-A1-03/078662
- WO-A1-2004/074518
- WO-A2-99/15657
- TAKAHASHI N ET AL: "Expression of 53BP2 and ASPP2 proteins from TP53BP2 gene by alternative splicing", BIOCHEMICAL AND BIOPHYSICAL RESEARCH COMMUNICATIONS, ACADEMIC PRESS INC. ORLANDO, FL, US, Bd. 315, Nr. 2, 5. März 2004 (2004-03-05), Seiten 434-438, XP004487296, ISSN: 0006-291X, DOI: 10.1016/J.BBRC.2004.01.079
- TRIGIANTE GIUSEPPE ET AL: "ASPP [corrected] and cancer", NATURE REVIEWS. CANCER, NATUR PUBLISHING GROUP, LONDON, GB, Bd. 6, Nr. 3, 1. März 2006 (2006-03-01), Seiten 217-226, XP002610068, ISSN: 1474-175X, DOI: 10.1038/NRC1818
- KAMPA KERSTIN M ET AL: "Apoptosis-stimulating protein of p53 (ASPP2) heterozygous mice are tumor-prone and have attenuated cellular damage-response thresholds.", PROCEEDINGS OF THE NATIONAL ACADEMY OF SCIENCES OF THE UNITED STATES OF AMERICA 17 MAR 2009 LNKD- PUBMED:19251665, Bd. 106, Nr. 11, 17. März 2009 (2009-03-17), Seiten 4390-4395, XP002661058, ISSN: 1091-6490
- VIVES VIRGINIE ET AL: "ASPP2 - A gene that controls life and death in vivo", CELL CYCLE, Bd. 5, Nr. 19, Oktober 2006 (2006-10), Seiten 2187-2190, XP002661059,
- KAMPA KERSTIN M ET AL: "New insights into the expanding complexity of the tumor suppressor ASPP2", CELL CYCLE, XX, GEORGETOWN, TX, US, Bd. 8, Nr. 18, 15. September 2009 (2009-09-15), Seiten 2871-2876, XP008144062, ISSN: 1538-4101, DOI: 10.4161/CC.8.18.9474

### SEQUENZ PROTOKOLL

<110> Eberhard-Karls-Universität Tübingen, Universitätsklinikum
<120> ASPP2 -Splicevariante
<130> 5402P442WO
<160> 3
<170> PatentIn version 3.3
<210> 1
   <211> 879
   <212> PRT
   <213> Homo sapiens
<400> 1
   Met Asp Leu Thr Leu Ala Glu Leu Gln Glu Met Ala Ser Arg Gln Gln
   1               5                   10                  15
   Gln Gln Ile Glu Ala Gln Gln Gln Leu Leu Ala Thr Lys Glu Gln Arg
               20                  25                  30
   Leu Lys Phe Leu Lys Gln Gln Asp Gln Arg Gln Gln Gln Gln Val Ala
           35                  40                  45
   Glu Gln Glu Lys Leu Lys Arg Leu Lys Glu Ile Ala Glu Asn Gln Glu
       50                  55                  60
   Ala Lys Leu Lys Lys Val Arg Ala Leu Lys Gly His Val Glu Gln Lys
   65                  70                  75                  80
   Arg Leu Ser Asn Gly Lys Leu Val Glu Glu Ile Glu Gln Met Asn Asn
                   85                  90                  95
   Leu Phe Gln Gln Lys Gln Arg Glu Leu Val Leu Ala Val Ser Lys Val
               100                 105                 110
   Glu Glu Leu Thr Arg Gln Leu Glu Met Leu Lys Asn Gly Arg Ile Asp
           115                 120                 125
   Ser His His Asp Asn Gln Ser Ala Val Ala Glu Leu Asp Arg Leu Tyr
       130                 135                 140
   Lys Glu Leu Gln Leu Arg Asn Lys Leu Asn Gln Glu Gln Asn Ala Lys
   145                 150                 155                 160
   Leu Gln Gln Gln Arg Glu Cys Leu Asn Lys Arg Asn Ser Glu Val Ala
                   165                 170                 175
   Val Met Asp Lys Arg Val Asn Glu Leu Arg Asp Arg Leu Trp Lys Lys
   180 185 190
   Lys Ala Ala Leu Gln Gln Lys Glu Asn Leu Pro Val Ser Ser Asp Gly
   195 200 205
   Asn Leu Pro Gln Gln Ala Ala Ser Ala Pro Ser Arg Val Ala Ala Val
   210 215 220
   Gly Pro Tyr Ile Gln Ser Ser Thr Met Pro Arg Met Pro Ser Arg Pro
   225 230 235 240
   Glu Leu Leu Val Lys Pro Ala Leu Pro Asp Gly Ser Leu Val Ile Gln
   245 250 255
   Ala Ser Glu Gly Pro Met Lys Ile Gln Thr Leu Pro Asn Met Arg Ser
   260 265 270
   Gly Ala Ala Ser Gln Thr Lys Gly Ser Lys Ile His Pro Val Gly Pro
   275 280 285
   Asp Trp Ser Pro Ser Asn Ala Asp Leu Phe Pro Ser Gln Gly Ser Ala
   290 295 300
   Ser Val Pro Gln Ser Thr Gly Asn Ala Leu Asp Gln Val Asp Asp Gly
   305 310 315 320
   Glu Val Pro Leu Arg Glu Lys Glu Lys Lys Val Arg Pro Phe Ser Met
   325 330 335
   Phe Asp Ala Val Asp Gln Ser Asn Ala Pro Pro Ser Phe Gly Thr Leu
   340 345 350
   Arg Lys Asn Gln Ser Ser Glu Asp Ile Leu Arg Asp Ala Gln Val Ala
   355 360 365
   Asn Lys Asn Val Ala Lys Val Pro Pro Pro Val Pro Thr Lys Pro Lys
   370 375 380
   Gln Ile Asn Leu Pro Tyr Phe Gly Gln Thr Asn Gln Pro Pro Ser Asp
   385 390 395 400
   Ile Lys Pro Asp Gly Ser Ser Gln Gln Leu Ser Thr Val Val Pro Ser
   405 410 415
   Met Gly Thr Lys Pro Lys Pro Ala Gly Gln Gln Pro Arg Val Leu Leu
   420 425 430
   Ser Pro Ser Ile Pro Ser Val Gly Gln Asp Gln Thr Leu Ser Pro Gly
   435 440 445
   Ser Lys Gln Glu Ser Pro Pro Ala Ala Ala Val Arg Pro Phe Thr Pro
   450 455 460
   Gln Pro Ser Lys Asp Thr Leu Leu Pro Pro Phe Arg Lys Pro Gln Thr
   465 470 475 480
   Val Ala Ala Ser Ser Ile Tyr Ser Met Tyr Thr Gln Gln Gln Ala Pro
   485 490 495
   Gly Lys Asn Phe Gln Gln Ala Val Gln Ser Ala Leu Thr Lys Thr His
   500 505 510
   Thr Arg Gly Pro His Phe Ser Ser Val Tyr Gly Lys Pro Val Ile Ala
   515 520 525
   Ala Ala Gln Asn Gln Gln Gln His Pro Glu Asn Ile Tyr Ser Asn Ser
   530 535 540
   Gln Gly Lys Pro Gly Ser Pro Glu Pro Glu Thr Glu Pro Val Ser Ser
   545 550 555 560
   Val Gln Glu Asn His Glu Asn Glu Arg Ile Pro Arg Pro Leu Ser Pro
   565 570 575
   Thr Lys Leu Leu Pro Phe Leu Ser Asn Pro Tyr Arg Asn Gln Ser Asp
   580 585 590
   Ala Asp Leu Glu Ala Leu Arg Lys Lys Leu Ser Asn Ala Pro Arg Pro
   595 600 605
   Leu Lys Lys Arg Ser Ser Ile Thr Glu Pro Glu Gly Pro Asn Gly Pro
   610 615 620
   Asn Ile Gln Lys Leu Leu Tyr Gln Arg Thr Thr Ile Ala Ala Met Glu
   625 630 635 640
   Thr Ile Ser Val Pro Ser Tyr Pro Ser Lys Ser Ala Ser Val Thr Ala
   645 650 655
   Ser Ser Glu Ser Pro Val Glu Ile Gln Asn Pro Tyr Leu His Val Glu
   660 665 670
   Pro Glu Lys Glu Val Val Ser Leu Val Pro Glu Ser Leu Ser Pro Glu
   675 680 685
   Asp Val Gly Asn Ala Ser Thr Glu Asn Ser Asp Met Pro Ala Pro Ser
   690 695 700
   Pro Gly Leu Asp Tyr Glu Pro Glu Gly Val Pro Asp Asn Ser Pro Asn
   705 710 715 720
   Leu Gln Asn Asn Pro Glu Glu Pro Asn Pro Glu Ala Pro His Val Leu
   725 730 735
   Asp Val Tyr Leu Glu Glu Tyr Pro Pro Tyr Pro Pro Pro Pro Tyr Pro
   740 745 750
   Ser Gly Glu Pro Glu Gly Pro Gly Glu Asp Ser Val Ser Met Arg Pro
   755 760 765
   Pro Glu Ile Thr Gly Gln Val Ser Leu Pro Pro Gly Lys Arg Thr Asn
   770 775 780
   Leu Arg Lys Thr Gly Ser Glu Arg Ile Ala His Gly Met Arg Val Lys
   785 790 795 800
   Phe Asn Pro Leu Ala Leu Leu Leu Asp Ser Ser Leu Glu Gly Glu Phe
   805 810 815
   Asp Leu Val Gln Arg Ile Ile Tyr Glu Val Asp Asp Pro Ser Leu Pro
   820 825 830
   Asn Asp Glu Gly Ile Thr Ala Leu His Asn Ala Val Cys Ala Gly His
   835 840 845
   Thr Glu Ile Val Lys Phe Leu Val Gln Phe Gly Val Asn Val Asn Ala
   850 855 860
   Ala Asp Ser Asp Gly Trp Ser Ser Gly Glu Asp Gly His Asn Glu
   865 870 875
<210> 2
   <211> 9
   <212> PRT
   <213> Homo sapiens
<400> 2
   Ser Ser Gly Glu Asp Gly His Asn Glu
   1 5
<210> 3
   <211> 3451
   <212> DNA
   <213> Homo sapiens
<400> 3
   gctggggccc gacccgggat tagttggttt cggagcggag gagggagccc cgaccgtcac 60
   gagcgtcgaa gagacaaagc cgcgtcaggg ggcccggccg gggcggggga gcccggggct 120
   tgttggtgcc ccagcccgcg cggagggccc ttcggacccg cgcgccgccg ctgccgccgc 180
   cgccgcctcg caacaggtcc gggcggcctc gctctccgct cccctccccc gcatccgcga 240
   ccctccgggg cacctcagct cggccggggc cgcagtctgg ccacccgctt ccatgcggtt 300
   cgggtccaag atgatgccga tgtttcttac cgtgtatctc agtaacaatg agcagcactt 360
   cacagaagtt ccagttactc cagaaacaat atgcagagac gtggtggatc tgtgcaaaga 420
   acccggcgag agtgattgcc atttggctga agtgtggtgt ggctctgtag agatagagtt 480
   tcatcatgtt ggccaggatg gtctcgatct cctgaccttg tgatccgcct gcctcggcct 540
   cccaaagtgc tggattacag gtgtgagcca ccacgatcag cctctagtgt ttaaaaaaga 600
   acgtccagtt gcggataatg agcgaatgtt tgatgttctt caacgatttg gaagtcagag 660
   gaacgaagtt cgcttcttcc ttcgtcatga acgcccccct ggcagggaca ttgtgagtgg 720
   accaagatct caggatccaa gtttaaaaag aaatggtgta aaagttcctg gtgaatatcg 780
   aagaaaggag aacggtgtta atagtcctag gatggatctg actcttgctg aacttcagga 840
   aatggcatct cgccagcagc aacagattga agcccagcaa caattgctgg caactaagga 900
   acagcgctta aagtttttga aacaacaaga tcagcgacaa cagcaacaag ttgctgagca 960
   ggagaaactt aaaaggctaa aagaaatagc tgagaatcag gaagctaagc taaaaaaagt 1020
   gagagcactt aaaggccacg tggaacagaa gagactaagc aatgggaaac ttgtggagga 1080
   aattgaacag atgaataatt tgttccagca aaaacagagg gagctcgtcc tggctgtgtc 1140
   aaaagtagaa gaactgacca ggcagctaga gatgctcaag aacggcagga tcgacagcca 1200
   ccatgacaat cagtctgcag tggctgagct tgatcgcctc tataaggagc tgcagctaag 1260
   aaacaaattg aatcaagagc agaatgccaa gctacaacaa cagagggagt gtttgaataa 1320
   gcgtaattca gaagtggcag tcatggataa gcgtgttaat gagctgaggg accggctgtg 1380
   gaagaagaag gcagctctac agcaaaaaga aaatctacca gtttcatctg atggaaatct 1440
   tccccagcaa gccgcgtcag ccccaagccg tgtggctgca gtaggtccct atatccagtc 1500
   gtctactatg cctcggatgc cctcaaggcc tgaattgctg gtgaagccag ccctgccgga 1560
   tggttccttg gtcattcagg cttcagaggg gccgatgaaa atacagacac tgcccaacat 1620
   gagatctggg gctgcttcac aaactaaagg ctctaaaatc catccagttg gccctgattg 1680
   gagtccttca aatgcagatc ttttcccaag ccaaggctct gcttctgtac ctcaaagcac 1740
   tgggaatgct ctggatcaag ttgatgatgg agaggttccg ctgagggaga aagagaagaa 1800
   agtgcgtccg ttctcaatgt ttgatgcagt agaccagtcc aatgccccac cttcctttgg   1860
   tactctgagg aagaaccaga gcagtgaaga tatcttgcgg gatgctcagg ttgcaaataa   1920
   aaatgtggct aaagtaccac ctcctgttcc tacaaaacca aaacagatta atttgcctta   1980
   ttttggacaa actaatcagc caccttcaga cattaagcca gacggaagtt ctcagcagtt   2040
   gtcaacagtt gttccgtcca tgggaactaa accaaaacca gcagggcagc agccgagagt   2100
   gctgctatct cccagcatac cttcggttgg ccaagaccag accctttctc caggttctaa    2160
   gcaagaaagt ccacctgctg ctgccgtccg gccctttact ccccagcctt ccaaagacac   2220
   cttacttcca cccttcagaa aaccccagac cgtggcagca agttcaatat attccatgta   2280
   tacgcaacag caggcgccag gaaaaaactt ccagcaggct gtgcagagcg cgttgaccaa   2340
   gactcatacc agagggccac acttttcaag tgtatatggt aagcctgtaa ttgctgctgc   2400
   ccagaatcaa cagcagcacc cagagaacat ttattccaat agccagggca agcctggcag   2460
   tccagaacct gaaacagagc ctgtttcttc agttcaggag aaccatgaaa acgaaagaat   2520
   tcctcggcca ctcagcccaa ctaaattact gcctttctta tctaatcctt accgaaacca   2580
   gagtgatgct gacctagaag ccttacgaaa gaaactgtct aacgcaccaa ggcctctaaa   2640
   gaaacgtagt tctattacag agccagaggg tcctaatggg ccaaatattc agaagctttt   2700
   atatcagagg accaccatag cggccatgga gaccatctct gtcccatcat acccatccaa   2760
   gtcagcttct gtgactgcca gctcagaaag cccagtagaa atccagaatc catatttaca   2820
   tgtggagccc gaaaaggagg tggtctctct ggttcctgaa tcattgtccc cagaggatgt   2880
   ggggaatgcc agtacagaga acagtgacat gccagctcct tctccaggcc ttgattatga   2940
   gcctgaggga gtcccagaca acagcccaaa tctccagaat aacccagaag aaccaaatcc   3000
   agaggctcca catgtgcttg atgtgtacct ggaggagtac cctccatacc cacccccacc    3060
   atacccatct ggggagcctg aagggcccgg agaagactcg gtgagcatgc gcccgcctga   3120
   aatcaccggg caggtctctc tgcctcctgg taaaaggaca aacttgcgta aaactggctc   3180
   agagcgtatc gctcatggaa tgagggtgaa attcaacccc cttgctttac tgctagattc   3240
   gtctttggag ggagaatttg accttgtaca gagaattatt tatgaggttg atgacccaag   3300
   cctccccaat gatgaaggca tcacggctct tcacaatgct gtgtgtgcag gccacacaga   3360
   aatcgttaag ttcctggtac agtttggtgt aaatgtaaat gctgctgata gtgatggatg   3420
   gagttcagga gaagatgggc ataatgaata a                                  3451

## Beschreibung

Die vorliegende Erfindung betrifft eine neue ASPP2-Splicevariante, insbesondere eine Nukleinsäuresequenz und eine hiervon abgeleitete Aminosäuresequenz, zur Verwendung als Marker zur Diagnose von Krebs.

Unter dem Begriff "Krebs" werden im Allgemeinen sowohl solide Tumore als auch bösartige Hämoblastosen zusammengefasst. Nachdem Krebs nach den Erkrankungen des Herz-Kreislauf-Systems in den meisten Industrieländern die zweithäufigste Todesursache ist, kommt der Diagnose und Behandlung von Krebs eine sehr hohe medizinische, wirtschaftliche sowie auch soziale und sozialpolitische Bedeutung zu.

Krebserkrankungen haben eine vielfältige und häufig multifaktoriell bedingte Ethologie. Sie können alle Organe des menschlichen Körpers betreffen bzw. aus den entsprechenden Geweben entstehen. Demzufolge sind Krebserkrankungen auch auf zellulärer Ebene sehr heterogen, was sich häufig erschwerend auf ihre Diagnose auswirkt.

Bezüglich der Gewebemorphologie unterscheidet man bei Krebserkrankungen zwischen soliden Tumoren und, wie bei Hämoblastosen, voneinander separierten Einzelzellen.

Eine der häufigsten Hämoblastosen ist die akute myeloische Leukämie. Hierbei handelt es sich um eine Erkrankung des blutbildenden Systems, die zur massiven Vermehrung unreifer Blutzellvorstufen im Knochenmark sowie häufig auch im Blut führt.

Während die Behandlung solider Tumore in der überwiegenden Zahl der Fälle mittels operativer Entfernung in Kombination mit Bestrahlung therapiert wird, werden Hämoblastosen wie die akute myeloische Leukämie durch Chemotherapie, ggf. unterstützt durch Transplantation von Knochenmark, behandelt.

Ein bekanntes Problem bei der Chemotherapie akuter myeloischer Leukämien ist das zum Teil sehr unterschiedliche Ansprechen auf die Chemotherapie. So ist bei einem bestimmten Anteil der Patienten eine gesteigerte Therapierefrakterität gegenüber Chemotherapeutika festzustellen. Diese geht mit einer deutlich verschlechterten Heilungsprognose einher.

Ein weiteres Problem, das Krebsarten mit soliden Tumoren und Hämoblastosen gleichermaßen betrifft, ist das Problem der minimalen Resterkrankungen. Bei der Therapie durch operatives Entfernen, Bestrahlung oder Chemotherapie können einzelne Krebszellen im Körper des Patienten verbleiben und ggf. nach einer gewissen Latenzzeit zu einer erneuten Krebserkrankung führen. Da solche minimale Resterkrankungen den Therapieerfolg insgesamt zunichte maschen, gilt auch ihrer Erkennung in der Krebsdiagnostik erhebliche Aufmerksamkeit.

Eine solche Diagnostik basiert, wie auch bei der allgemeinen Krebsdiagnostik, zumeist auf DNA oder RNA bestimmenden Tests, mit deren Hilfe genetische Aberrationen festgestellt werden können, die für die Entstehung von Krebs bzw. für Krebszellen charakteristisch sind. Weiterhin werden auch immunologische Tests verwendet, mit deren Hilfe krebszellspezifische Marker auf der Zelloberfläche oder im Cytoplasma nachgewiesen werden können. Andere in der Krebsdiagnostik angewandte Verfahren basieren auf der Feststellung aberranter Transkriptionsprofile von Krebszellen im Verhältnis zu gesunden Zellen des Herkunftsgewebes.

Solche Diagnoseverfahren können zu einer gezielten Therapie führen, wenn die geänderten Transkriptionsprofile Aufschluss über die Ethologie bzw. Genetik der individuellen Krebserkrankung zulassen.

Im Rahmen einer derartigen Diagnose werden verschiedene Marker bezüglich ihres Vorhandenseins oder Nichtvorhandenseins bzw. ihrer intracellulären Konzentration gemessen. In der Krebs-Diagnostik werden dabei sowohl Nukleinsäure-als auch Aminosäuresequenzen als Marker verwendet, die über geeignete Verfahren nachgewiesen werden können. Auf genetischer Ebene lassen sich die Marker durch spezifische Amplifikation und/oder Bindung von markierten Sonden nachweisen, während auf der Proteinebene der Nachweis beispielsweise über spezifisch bindende Antikörper erfolgt.

Beispielsweise beschreibt die US2002/0086384 A1 eine Reihe von Transkripten und Proteinen, die durch alternatives Splicing entstehen und die im Rahmen einer Krebsdiagnostik als Marker dienen können.
Takahashi et al., "Expression of 53BP2 and ASPP2 proteins from TP53BP2 gene by alternative Splicing," Biochemical and Biophysical Research Communications 315 (2004), 434-438, beschreiben die Expression zweier Proteine, 53BP2 und ASPP2, ausgehend vom Gen TP53BP2, welche durch unterschiedliches Splicing entstehen.
Ferner beschreiben Trigante und Liu, "ASPPS and Cancer", Nature Reviews, Cancer, 6 (2006) 217-226, den engen Zusammenhang der apoptotischen Funktion von p53 und dessen Tumor-Suppressor-Funktion, und diskutieren das Zusammenspiel zwischen p53 und ASPP-Familienmitgliedern, die u.a. als spezifische Regulatoren der p53-vermittelten Apoptose gelten.
Ferner offenbart die WO 03/078662 A1 ein Verfahren zur Vorhersage des Verlaufs einer Krebserkrankung, bei welchem der Expressionslevel von u.a. p53BP2 gemessen und in Relation zu Kontrollproben von Nicht-Erkrankten gesetzt wird.

Viele der bekannten Marker erlauben für sich genommen jedoch keine zuverlässige Diagnose. Es besteht somit ein Bedarf an zusätzlichen, zuverlässigen Markern zur sicheren Diagnose von Krebs.

Vor diesem Hintergrund liegt der vorliegenden Erfindung die Aufgabe zugrunde, Marker bereitzustellen, die eine zuverlässige Krebserkennung ermöglichen und die darüber hinaus möglichst die Gewinnung von weiteren Daten über das Transkriptionsprofil der betreffenden Zellen ermöglichen, die für die Behandlung der individuellen Krebserkrankung hilfreich sind.

Diese und andere Aufgaben werden erfindungsgemäß gelöst durch ein Verfahren zur in vitro Bestimmung des Vorliegens oder einer Prädisposition eines Patienten für die Entwicklung von Krebs, wobei in einer biologischen Probe des Patienten das Vorliegen eines Markers bestimmt wird, wobei der Marker eine Splice-Variante von ASPP2 ist, und wobei Marker der ausgewählt ist aus
a) einer Aminosäuresequenz, die die Aminosäuresequenz SEQ ID Nr. 2 aus dem beigefügten Sequenzprotokoll umfasst, oder
b) einer Nukleinsäure, die eine Nukleinsäure umfasst, die für die Aminosäuresequenz mit der SEQ ID Nr. 2 codiert.

Dabei ist insbesondere bevorzugt, wenn der Marker eine ASPP2 Isoform ist, die im Vergleich zu den natürlich vorkommenden ASPP2 Isoformen im C-terminalen Bereich verkürzt ist und die die Aminosäuresequenz mit der SEQ ID Nr. 2 enthält, sowie ferner insbesondere, wenn der Marker ausgewählt ist aus
a) der Aminosäuresequenz mit der SEQ ID Nr. 2;
b) einer für die Aminosäuresequenz mit der SEQ ID Nr. 2 codierenden Nukleinsäure.

Der Krebs, der mittels des erfindungsgemäßen Verfahrens diagnostiziert oder vorhergesagt werden kann, ist dabei ausgewählt aus hämatologischen Neoplasien, insbesondere aus Leukämien (wie akute myeloische und lymphatische Leukämie sowie chronischer myeloischer Leukämie), soliden Tumoren sowie Präkanzerosen.

Entsprechend betrifft die Erfindung auch eine Aminosäuresequenz, umfassend die mit der SEQ ID Nr. 2 wieder gegebene Aminosäuresequenz zur Verwendung als Marker zur Diagnose von Krebs.

ASPP2 steht in diesem Zusammenhang für "Apoptose stimulierendes Protein von p53" und wird auch als TP53BP2 (tumor protein p53 binding protein, 2) oder 53BP2, BBP, P53BP2 oder PPP1R13A (NCBI Genbank GeneID 7159) bezeichnet. Über direkte Bindung an p53 triggert bzw. verstärkt ASPP2 nach Zellstress die Induktion von programmierten Zelltodmechanismen, also der Apoptose. Zum einen ist nach Erkenntnissen der Erfinder damit eine erhöhte Tumortherapierefraktärität assoziiert. Weiter führt im Mausmodell eine erniedrigte ASPP2-Expression durch das fehlende Vermögen einer geregelten Apoptoseinduktion nach Zellschädigung zu einer erhöhten Tumorinzidenz. Passend hierzu konnte in vielen humanen Krebsarten eine ASPP2 Suppression dokumentiert werden. Eine niedrige Expression von ASPP2 konnte dementsprechend mit erhöhter Tumorinzidenz, schlechterem überlegen und schlechterem Ansprechen auf Therapien korreliert werden.

Die Erfinder konnten nun in ca. 50% der von ihnen untersuchten Patienten mit akuter myeloischer Leukämie eine bis dato unbekannte Splicevariante des Tumorsuppressorgens ASPP2 identifizieren, im Folgenden als ASPP2 Transkriptvariante κ bezeichnet. Diese kodiert für eine ASPP2 Protein-Variante, im Folgenden als ASPP2 Isoform κ bezeichnet, bei der im Vergleich zu den bekannten ASPP2 ProteinVarianten große Teile des C-Terminus mit den dort vorhandenen p53 Bindungsstellen deletiert sind. ASPP2 Isoform κ hat folglich die Fähigkeit verloren nach Zellstress/-schädigung über p53 Apoptose zu induzieren.

Erste weitere Ergebnisse zeigen, dass sowohl ASPP2 Transkriptvariante κ als auch ASPP2 Isoform κ auch in anderen Leukämieformen, Lymphomen und soliden Tumoren vorkommt.
konnte dementsprechend mit erhöhter Tumorinzidenz, schlechterem Überleben und schlechterem Ansprechen auf Therapien korreliert werden.

Die Erfinder konnten nun in ca. 50% der von ihnen untersuchten Patienten mit akuter myeloischer Leukämie eine bis dato unbekannte Splicevariante des Tumorsuppressorgens ASPP2 identifizieren, im Folgenden als ASPP2 Transkriptvariante κ bezeichnet. Diese kodiert für eine ASPP2 Protein-Variante, im Folgenden als ASPP2 Isoform κ bezeichnet, bei der im Vergleich zu den bekannten ASPP2 ProteinVarianten große Teile des C-Terminus mit den dort vorhandenen p53 Bindungsstellen deletiert sind. ASPP2 Isoform κ hat folglich die Fähigkeit verloren nach Zellstress/-schädigung über p53 Apoptose zu induzieren.

Erste weitere Ergebnisse zeigen, dass sowohl ASPP2 Transkriptvariante κ als auch ASPP2 Isoform κ auch in anderen Leukämieformen, Lymphomen und soliden Tumoren vorkommt.

Nach Erkenntnissen der Erfinder kann die Transkriptvariante dabei sowohl auf RNA-Ebene, d.h. durch alternatives Spicing, generiert worden sein, oder aber bereits im Genom des Betroffenen manifestiert sein, d.h., dass die Information für die Transkritpvariante bereits genetisch in der DNA des Betroffenen festgelegt ist. Daher kann erfindungsgemäß ein Nachweis des Markers auch auf DNA-Ebene stattfinden, bei welchem in der genetischen Information des zu Untersuchenden die für den Marker codierenden Nukleinsäuresequenzen detektiert werden.

Nach Erkenntnis der Erfinder eignen sich die genomische Sequenz, die Transkripte und Translationsprodukte dieser neuen Splicevariante damit ausgezeichnet als Marker für die Krebsdiagnostik. Durch Nachweis dieser Marker lassen sich folglich Patienten und Patienterigruppen mit erhöhtem Malignomrisiko identifizieren und Aussagen über Sterblichkeit und Therapieansprechen generieren. Ferner sind diese Marker ideal für das Therapiemonitoring geeignet.

Dementsprechend wird erfindungsgemäß unter einer für die Aminosäuresequenz mit der SEQ ID Nr. 2 codierenden Nukleinsäure u.a. mRNA, cDNA bzw. die mRNA-codierende Nukleinsaeure (gDNA) verstanden.

Da die erfindungsgemäße, ASPP2 Transkriptvariante κ bzw. das hiervon translatierte Protein keine Apoptose über p53 induzieren kann, sind die auf die neuen Marker positiv getesteten Patienten anfällig für eine gesteigerte Therapierefrakterität gegenüber zytostatischen/toxischen Therapeutika. Bei dieser Patientengruppe bieten sich daher spezielle Therapien an, um durch Wiederherstellung der "üblichen" ASPP2-Aktivität Tumorzellen in den programmierten Zelltod zu treiben und/oder die Sensitivität gegenüber anderen Therapeutika (wieder) zu verbessern.

In diesem Zusammenhang kann die Wiederherstellung der ASSP2-Wildtypexpression durch Stimulation der Transkription, wie z.B. durch demethylierende Substanzen oder durch Gentransfer oder eine Supplementierung von Wildtyp-ASSP2 genauso sinnvoll sein wie die Simulation der ASSP2-Wildtypfunktion durch dem Patienten verabreichte Antikörper oder Small Molecules, die an p53 die ASPP2 Bindungsstellen aktivieren bzw. die ASPP2-p53 Bindungsstellen mimikrieren. Erfindungsgemäß betrifft die vorliegende Erfindung demnach auch den Einsatz solcher Mittel, um die ASSP2-Wildtypexpression wiederherzustellen.

Die neuen Marker bieten also neben der reinen Diagnostik auch Vorteile im Zusammenhang mit einer verbesserten Therapie, die sich an die Diagnostik anschließen kann.

Die besondere diagnostische und therapeutische Relevanz der neuen Marker lässt sich wie folgt erklären, wobei die Erfinder nicht auf diese Erklärung festgelegt sein wollen.

ASPP2 gehört zur Familie der ASPP-Proteine, die im Jahre 1994 erstmals beschrieben wurde; Iwabushi, K. et al. (1994): Two proteins that bind to wild-type but not mutant p53 Proc. Natl. Acad. Sci. USA 91: 6098-6102. In Säugetieren sind insgesamt drei Mitglieder der ASPP-Familie bekannt, ASPP1, ASPP2 und das evolutionär höchst konservierte iASPP. Alle drei Proteine weisen hohe Sequenzhomologie im Bereich ihres C-Terminus auf, in welchem in erster Linie Bindestellen für ASPP-Bindepartner, beispielsweise das Protein p53 und bcl-2, vorhanden sind; Gorina, S und Pavelitch, NP (1996): Structure of the p53 Tumor Suppressor bound to the anchyrin and SH3 domains of 53BP2. Science 274: 1001-1005; Naumovski, L und Cleary, ML (1996): The p53 binding protein 53BP2 also interacts with bcl2 and impedes cell cycle progression at G2/M. Mol. Cell. Biol. 16: 3884-3892.

Die bislang am besten charakterisierte Funktion der ASSP-Proteinfamilie betrifft ihre Fähigkeit, p53-induzierte Apoptose zu regulieren. ASPP2 verstärkt hierbei die Fähigkeit von p53, spezifisch die Expression proapoptischer Zielgene zu stimulieren; Samuels-Lev, Y et al. (2001): ASPP proteins specifically stimulate the apoptotic function of p53. Mol. Cell. 8: 781-794; Bergamaschi, D et al. (2004) ASPP1 and ASPP2: Common Activators of p53 Family Members. Mol. Cell. Biol. 24: 1341-1350.

Im Gegensatz hierzu scheint das iASPP-Protein eine für die Funktion von ASPP1 und/oder ASPP2 inhibitorische Funktion auszuführen, indem es kompetitiv die ASPP-Bindestellen an beispielsweise p53 bindet und somit die Funktion von ASPP1 und ASPP2 hemmt; Bergamaschi, D et al. (2003): iASPP oncoprotein is a key inhibitor of p53 conserved from worm to human. Nat. Genet. 33: 162-167.

In diesem Zusammenhang weist eine Reihe experimenteller Ergebnisse darauf hin, dass der haploide Tumorsuppressor ASPP2 im Regulationsnetzwerk von p53 induzierter Apoptose eine wichtige Rolle spielt, und bei einer Reihe relevanter Krebserkrankungen lässt sich auch eine verminderte Expression von ASPP2 nachweisen. So konnte gezeigt werden, dass ASPP2 heterozygote Mäuse ein gegenüber homozygoten Wildtypmäusen deutlich erhöhtes Auftreten von Tumoren im Verlaufe ihrer Lebensspanne aufweisen; Kampa KM et al. (2009): Apoptosis stimulating protein of p53 (ASPP2) heterozygous mice are tumor-prone and have attenuated cellular damage-response thresholds. Proc. Natl. Acad. Sci. USA 106(11):4390-4395; Vives V et al. (2006): ASPP2 is a haploinsufficient tumor suppressor that cooperates with p53 to suppress tumor growth. Genes and Development 20:1262-1267.

Ausgehend von dem ASPP2-Gen, welches insgesamt 19 Exons umfasst, entstehen durch alternatives Splicing verschiedene Transkripte. So werden in der Datenbank (Genbank) des National Center for Biotechnology Information (NCBI) zwei Transkripte des ASPP2-Gens geführt, die ASPP2 Transkriptvariante 1 (Datenbank-Nr. NM_001031685) und die ASPP2 Transkriptvariante 2 (Datenbank-Nr. NM_005426). Die Nummerierung der Exons entspricht im Folgenden der Nummerierung gemäß den obengenannten Genbank-Einträgen zu ASPP2 Transkriptvarianten 1 und 2.

Die ASPP2 Transkriptvariante 2 umfasst alle Exons 1 bis 19 des ASPP2-Gens und weist eine Länge von 3405 bp auf (nach NCBI Consensus CDS [CCDS] Project, CCDS ID: CCDS44319.1). Im Gegensatz hierzu ist bei der ASPP2 Transkriptvariante 1 das Exon 3 durch Splicing entfernt, womit sich eine gegenüber Transkriptvariante 2 verkürzte Sequenz von insgesamt 3018 bp (CCDS ID: CCDS1538.1) ergibt.

Beide Transkripte kodieren für Isoformen des ASPP2-Proteins, Transkriptvariante 1 für Isoform 1 und Transkriptvariante 2 für Isoform 2.

ASSP2 Isoform 1 (Datenbank-Nr. NP_001026855) ist mit 1134 Aminosäuren (aa) das größere der beiden Proteine. ASPP2 Isoform 2 (Datenbank-Nr. NP_005417) stellt, trotz Integration von Exon 3 in Transkriptvariante 2, eine N-terminal trunkierte Isoform (mit alternativem im Vergleich zu Isoform 1 weiter C-terminal gelegenem Startcodon) von 1005 aa Länge dar, die jedoch ansonsten sequenzidentisch mit Isoform 1 ist.

Wie bereits weiter oben erwähnt, finden sich im C-terminalen Bereich die für die Bindung an bekannte Bindungspartner, wie bspw. p53 oder bcl-2, notwendigen Bindedomänen.

Beide bekannten Isoformen werden grundsätzlich in gesundem wie Tumor-Gewebe exprimiert. Bei einzelnen Erkrankungen kann eine Repression des ASPP2-Gens in den Krebszellen vorliegen.

Wie bereits erwähnt, haben die Erfinder der vorliegenden Anmeldung nun in eigenen Untersuchungen festgestellt, dass neben den bekannten Transkriptvarianten überraschenderweise eine weitere Transkriptvariante existiert, die in Tumorgeweben nachweisbar exprimiert wird.

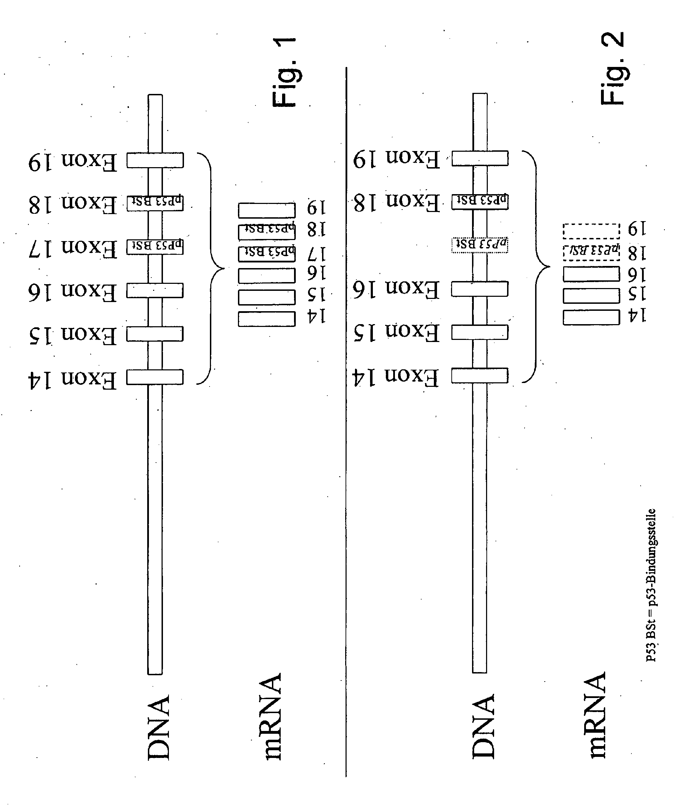

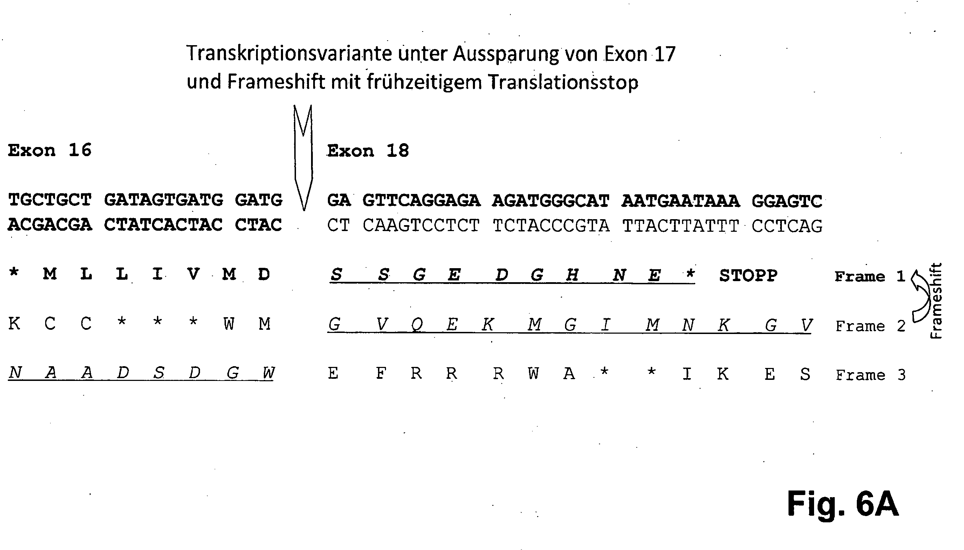

Diese Transkriptvariante entsteht, wie in Fig. 1 gezeigt, durch Fehl-Splicing mit Auslassung des gesamten Exon 17. Durch dieses Fehl-Splicing erfolgt die Transkription von Exon 18 direkt nach Exon 16. Hieraus resultiert eine Leserasterverschieburig mit deutlich verkürztem Transkript mit einer für diese Variante charakteristischen Sequenz (siehe Fig. 6A; sowie Seq. ID Nr. 1 und 2 aus dem beigefügten Sequenzprotokoll).

Diese Leserasterverschiebung wiederum bewirkt, dass das durch das betreffende Transkript kodierte Protein, im weiteren ASPP2κ (kappa) bezeichnet, gegenüber dem ASPP2 Wildtyp einen veränderten C-Terminus aufweist. Entsprechend der N-terminal differentiell codierten Transkriptionsvarianten ASPP2 Isoform 1 und Isoform 2 finden sich auch für ASPP2κ entsprechende Isoformen (ASPP2κ Isoform 1 und ASPP2κ Isoform 2). Darüberhinaus existiert nach Erkenntnissen der Erfinder noch mindestens eine weitere N-terminal trunkierte ASPP2κ Isoform.

ASPP2κ verfügt, wie bereits erwähnt, über eine für das ASPP2 Isoform κ-Protein einzigartige Sequenz von 9 Aminosäuren Länge (Seq. ID Nr. 2 aus dem beiliegenden Sequenzprotokoll) und ist darüber hinaus gegenüber den bekannten Proteinvarianten stark verkürzt. Diese Verkürzung, wie auch die Leserasterverschiebung, betrifft insbesondere den oben erwähnten Teil der bekannten ASPP2-Isoformen, in dem sich die Bindungsstellen für die Bindung der ASPP2-Proteine an bspw. p53 oder bcl-2 befinden, wie in Fig. 2 dargestellt.

Folglich handelt es sich bei ASPP2 Isoform κ um ein Protein, welches im Gegensatz zu den auch in gesundem Gewebe vorkommenden ASPP2 Isoformen 1 und 2 keine Bindeaktivität an p53 entfalten kann.

Mit der Bildung des ASPP2 Transkriptvariante κ im Zuge eines alternativen Splice-Vorganges geht in den Tumorzellen die Bildung der funktionell intakten ASPP2 Isoformen 1 und 2 ganz oder zumindest zu einem großen Teil verloren, was mit einer fehlenden oder zumindest stark verringerten Aktivität des ASPP2-Proteins in Tumorzellen verbunden ist. Mit anderen Worten, in den Tumorzellen erfolgt keine oder nur eine unzureichende Apoptose-Induktion über den p53-Signaltransduktionsweg.

Die Apoptose-Induktion stellt jedoch eine wichtige Grundlage für die (a) Aufrechterhaltung der Zell- und Gewebeintegrität nach Zellstress, als auch (b) für die Krebsbekämpfung mittels Chemotherapeutika dar.

Im Rahmen der Zellteilung (Mitose) kommt es stetig zu fehlerhaften Replikationen, was durch verschiedene Reparaturmechanismen in den allermeisten Fällen zuverlässig wieder behoben werden kann. Insbesondere nach Zellstress, wie Chemikalien (z.B. Benzole) oder radioaktiver bzw. UV-Strahlung, steigt die Zahl der Replikationsfehler an, und es besteht die Gefahr, dass Reparaturmechanismen versagen und fehlerhafte Nukleinsäuresequenzen an die Tochterzelle(n) weitergegeben werden - je nach betroffenem Gen kann dies einen ersten Schritt in der Onkogenese bedeuten. Als letzter Mechanismus im Rahmen der Reparaturmaschinerie dient der Zelle, zum Schutz des umgebenden Gewebes und letztendlich des Gesamtorganismus, die Selbstzerstörung durch Induktion des programmierten Zelltods (Apoptose). Eine fehlerhafte p53/ASPP2 Interaktion verhindert dies mit dem Ergebnis, daß fehlerhafte (mutagene) Nukleinsäuresequenzen im Rahmen der Mitose in die Tochterzellen weitervererbt werden können.

Trotz unterschiedlicher Ansätze, zumeist im Bereich der Zellteilungsmaschinerie, haben Chemotherapeutika gemeinsam, dass resultierend aus der induzierten Zellschädigung der programmierte Zelltod (Apoptose) eingeleitet wird. Die p53/ASPP2 Interaktion spielt dabei eine maßgebliche Rolle und die ASPP2-Funktionalität ist damit mitbestimmend für die Effizienz einer Chemotherapie.

ASPP2κ kann damit als Präkanzerose bzw. frühe Aberration im Rahmen der Tumorgenese verstanden werden.

Zudem ist bei ASPP2κ-positiven Krebserkrankungen von einer erhöhten Therapierefrakterität auszugehen.

Die neu gefundenen Transkript- bzw. Proteinvarianten von ASPP2 ermöglichen folglich eine völlig neue Art der Krebs-Diagnostik.

Zwar beschreibt die US 5,977,311 Proteinkomplexe, die ein ASPP2-Protein aufweisen, sowie Verfahren zur Diagnose und Behandlungsverfahren auf Basis dieser Proteinkomplexe, die bekannte Diagnostik beschränkt sich jedoch auf die in jedem Körpergewebe vorzufindenden bekannten Isoformen des ASPP2-Proteins.

Gleiches gilt für die US 7,081,340, welche ein Verfahren zur Diagnose von Krebs betrifft, bei dem die Konzentration einer Reihe von Transkripten, unter anderem von ASPP2, in einer biologischen Probe bestimmt wird. Auch hier findet sich kein Hinweis auf die von den Erfindern der vorliegenden Erfindung beschriebenen aberranten Splicevariante.

Im Gegensatz dazu ermöglichen die von den Erfindern der vorliegenden Anmeldung beschriebenen Transkript- und Proteinvarianten von ASPP2 eine einfache, spezifische Differenzierung nicht nur zwischen Tumor und normalem Gewebe, sondern auch zwischen Tumorgeweben verschiedener funktioneller Eigenschaften.

Die diagnostische Erfassung und Quantifizierung der ASPP2κ-Genprodukte innerhalb einer Zellpopulation liefert demnach nicht nur Informationen für die Krebsdiagnose an sich, sondern auch wichtige Hinweise für die Therapie und Prognose einer individuellen Krebserkrankung.

Erfindungsgemäß kann dabei auch eine Aminosäure als Marker eingesetzt werden, die sich N-terminal als Fusionsstelle an die Aminosäuresequenz SEQ ID Nr.2 anschließt, oder eine Nukleinsäure, die eine Nukleinsäure umfasst, die die N-terminal gelegene Fusionsstelle der Aminosäuresequenz mit der SEQ ID Nr. 2 umfasst.

Dabei stellt diese "Fusionsstelle" jeweils die Aminosäuresequenz dar, die aus der Transkriptvariante abgelesen wird, bei welcher Exon 17 fehlt, und bei der dadurch die Sequenz von Exon 18 direkt nach der Sequenz von Exon 16 abgelesen wird. Daher umfasst der erfindungsgemäße Marker auch Aminosäuresequenzen, die sich an solche Fusionsstellen anschließen.

Es versteht sich, dass das neue Verfahren zur Diagnose von Krebs nicht nur bei bereits an Krebs erkrankten Patienten, sondern auch bei Gesunden oder anderweitig erkrankten Personen angewendet werden kann. Auf diese Weise können Hinweise auf das individuelle Krebsrisiko eines Patienten und auf im Falle einer späteren Krebserkrankung notwendige Therapieansätze gewonnen werden. Insbesondere kann das Verfahren auch zur Diagnose von/bei potentiell Krebs-begünstigenden Erkrankungen eingesetzt werden.

Die der vorliegenden Erfindung zugrunde liegende Aufgabe wird auf diese Weise vollständig gelöst.

Im Rahmen der vorliegenden Anmeldung wird unter einer "Aminosäuresequenz" eine Sequenz verstanden, die aus zwei oder mehr Aminosäuren aufgebaut ist, wobei die Aminosäuren aus der Gruppe der zwanzig natürlich auftretenden Aminosäuren sowie der durch chemische Modifikation hieraus gewonnenen Derivate stammen. Die Aminosäuresequenz weist eine Primärstruktur, also die sequenzielle Abfolge von Aminosäuren auf, und kann Sekundärstrukturen, beispielsweise α-Helix oder β-Faltblatt, und Tertiärstrukturen bilden, wie beispielsweise in einem korrekt oder aberrant gefalteten Protein.

In diesem Zusammenhang kann eine der Primärstruktur nach identische Aminosäuresequenz durch Nukleinsäuresequenzen unterschiedlicher Sequenzabfolgen kodiert werden. Dies ist durch die Kodondegeneration bedingt, welche dazu führt, dass verschiedene Kodons (d.h. Nukleinsäuretriplets) für eine identische Aminosäure kodieren.

Unter einer deletierten Bindungsstelle wird vorliegend eine Bindungsstelle verstanden, die ihre Fähigkeit zum Binden ganz oder zumindest teilweise durch partielle oder vollständige Deletion verloren hat. Eine solche Deletion kann hierbei durch eine Deletion oder eine Leserasterverschiebung der Nukleinsäuresequenz, die der Aminosäuresequenz zugrunde liegt verursacht sein. Eine solche Leserasterverschiebung verursacht eine gegenüber dem unverschobenen Leseraster veränderte Kodierung der betroffenen Nukleinsäuresequenz, womit während der Translation der Nukleinsäuresequenz an dieser Stelle eine veränderte Aminosäuresequenz erzeugt wird.

Im Rahmen der vorliegenden Anmeldung wird unter "Nukleinsäuresequenz" insbesondere ein Makromolekül verstanden, welches als Sequenz von Desoxyribonukleinsäure- (DNA)-Nukleotiden oder Ribnokkleinsäure- (RNA)-Nukleotiden aufgebaut ist. Die zu bestimmenden Nukleinsäuren können in der Probe sowohl einzelsträngig als auch doppelsträngig vorliegen. Der Begriff schließt insbesondere - aber nicht darauf beschränkt - DNAs und RNAs mit ein, insbesondere mRNAs, microRNAs, rRNA und nicht kodierende RNAs sowie deren, durch reverse Transkription erzeugte cDNAs, sowie auch genomische DNA (einzelsträngig und doppelsträngig), und synthetische oder modifizierte Nukleinsäuren. Zudem umfasst der Begriff "Nukleinsäure" auch Aptamer-DNA und -RNA bzw. Fragmente genomischer DNA, die insbesondere auch methyliert sein kann.

Auch können diese Nukleotide eine Reihe chemischer Modifikationen aufweisen. Neben diesen natürlich vorkommenden Sequenzen sind jedoch vorliegend vom Begriff "Nukleinsäuresequenz" auch Sequenzen erfasst, die aus durch chemische Synthese gewonnenen Nukleotidanaloga oder Kombinationen mit der zuvor genannten Nukleotidtypen aufgebaut ist.

Beispiele für Nukleotidanaloga sind Peptidnukleotide, L-Nukleinsäuren oder Morpholinos. Entsprechende Syntheseverfahren für Mono- oder Polymere sind aus dein Stand der Technik bekannt.

Eine Nukleinsäuresequenz ist zunächst durch ihre Primärstruktur, also durch die sequenzielle Abfolge der Nukleotide innerhalb des Polymers bestimmt, sie kann aber auch Strukturen höherer Ordnung bilden, die sich durch die physikalische oder physikochemische Interaktion bestimmter Bereiche oder Nukleotide innerhalb der Sequenz oder zwischen voneinander getrennten Sequenzen ergeben. Beispiele für solche höheren Strukturen sind Hairpin-Loops oder Doppelstränge.

Es ist bevorzugt, wenn das erfindungsgemäße Verfahren folgende Schritte aufweist a) Bereitstellen einer biologischen Probe, b) Inkontaktbringen der biologischen Probe mit mindestens einem Bindereagenz, das einen erfindungsgemäßen Marker spezifisch bindet, c) Detektion mindestens eines in der biologischen Probe enthaltenen erfindungsgemäßen Markers, und d) Quantifizierung des mindestens einen Markers aus Schritt c).

Hierbei ist besonders bevorzugt, wenn zwischen den Schritten a) und b) ein oder mehrere Schritte zum Isolieren, Aufreinigen, Aufkonzentrieren und/oder chromatographischen Auftrennen von Nukleinsäuresequenzen oder Aminosäuresequenzen vorgesehen sind.

Mit dem erfindungsgemäßen Verfahren kann die Detektion der ASPP2κ Marker direkt in entsprechend gewonnen Gewebe- oder Zellproben vorgenommen werden. Entsprechende Verfahren, beispielsweise die in situ-Hybridisierung von Nukleinsäuresonden an in den Gewebe- oder Zellproben vorhandene Transkripte oder die Antikörperfärbung von Proteinen sind dem Fachmann umfangreich bekannt. Der Nachweis kann auch über an sich bekannte PCR-Verfahren erfolgen, in denen die erfindungsgemäßen Bindereagenzien als Sonden und/oder Primer eingesetzt werden.

Solche Verfahren ermöglichen eine Feststellung, ob entsprechende, durch die Nukleinsäuresonde oder den Antikörper spezifisch detektierte Transkripte oder Proteine in den Gewebe- oder Zellproben vorhanden sind, und erlauben über diese qualitative Aussage hinaus unter Umständen eine semiquantitative Analyse, in der der Grad der Expression bzw. Translation der betreffenden Marker mit anderen Markern korreliert wird.

Diese anderen Marker können beispielsweise andere Genprodukte von ASPP2 oder von Housekeeping-Genen sein, deren mRNA- bzw. Proteinkonzentration in den untersuchten Geweben bzw. Zellen bekannt ist.

Weitere Verfahren, die eine unter Umständen genauere quantitative Analyse eines Expressionsmusters bzw. von Proteinkonzentrationen zulassen, sind der Northern- oder Western-Blot oder die Verwendung von DNA- oder Protein-Mikroarrays. Solche Verfahren und Vorrichtungen sind aus dem Stand der Technik bekannt.

Das erfindungsgemäße Verfahren ermöglicht somit nicht nur die qualitative Aussage, ob überhaupt ASPP2k Marker in den Gewebe- bzw. Zellproben enthalten sind, sondern erlaubt auch eine semiquantitative oder gar quantitative Analyse der ASPP2κ Transkription und/oder Translation in den betreffenden Proben.

In diesem Zusammenhang ist es auch möglich, die über das erfindungsgemäße diagnostische Verfahren gewonnenen Informationen zu verwenden, um der Therapierefrakterität bei individuellen Krebserkrankungen gezielt entgegenzuwirken.

Dies kann im Falle einer ausschließlich auf der Dysfunktionalität der Isoform k basierende Funktionsverringerung von ASPP2 durch Supplementation der entsprechenden Transkriptvarianten 1 und 2 sowie ggf. der entsprechenden Proteine Isoform 1 und 2 erreicht werden. Daneben ist es natürlich auch möglich, eine stabile Supplementation mittels Gentherapie, also durch lokale oder systemische Infektion des Patienten mit einem Virus vorzunehmen, der eine nicht für inkorrektes Splicing anfällige ASPP2 Genvariante trägt.

Neben dem Verfahren zur in vitro Diagnose von Krebs betrifft die vorliegende Erfindung eine isolierte Aminosäuresequenz, die einer natürlich vorkommenden ASPP2 Isoform mit einem trunkierten C-Terminus und den hierin befindlichen p53-Bindungsstellen entspricht, und die die Sequenz mit der SEQ ID Nr. 2 aufweist. Insbesondere betrifft die vorliegende Erfindung eine isolierte Aminosäuresequenz, die aus einem Frameshift der ursprünglichen (Wildtyp-) Aminosäuresequenz resultiert, mit einem Exon 17 Splicing. Diese Translationsvariante trägt eine für sie charakteristische 9 Aminosäuren lange Sequenz am C-terminalen Ende und ist, wie bereits weiter oben erwähnt, im Vergleich zum Wildtyp stark verkürzt.

Die Aminosäuresequenz gemäß Seq. ID Nr. 2 entspricht der für ASPP2κ Isoformen spezifischen, 9 Aminosäuren langen Sequenz, die durch Translation des verschobenen Leserasters in der ASPP2 Transkriptvariante κ entsteht und am C-Terminus von ASPP2 Isoform κ lokalisiert ist.

Die Aminosäuresequenz mit der SEQ ID Nr. 1 zeigt die vollständige Aminosäuresequenz einer von den Erfindern identifizierten ASPP2κ Isoform, die insgesamt 879 Aminosäuren lang ist und ein Gesamtmolekulargewicht von ca. 97 kDa aufweist.

Mit der SEQ ID Nr. 3 ist die mRNA dieser ASPP2κ Isoform gezeigt, wobei die Basen Nr. 3422 bis 3448 für die für die Isoform spezifische Sequenz kodieren. Daher betrifft die Erfindung auch den Nachweis einer Nukleinsäuresequenz, die für die Aminosäuresequenz mit der SEQ ID Nr. 1 oder 2 codiert, also insbesondere der für die Isoform codierenden mRNA, die in SEQ ID Nr. 3 gezeigt ist, bzw. dem für die spezifische Sequenz codierenden Abschnitt dieser mRNA, d.h. die Basen Nr. 3422 bis 3448, oder einer genomischen Sequenz, die für die mRNA Nukleinsäuresequenz oder den Abschnitt codiert, sowie für eine Sequenz, die Sequenzen umfasst, die sich unmittelbar an die SEQ ID Nr. 2 anschließen.

Der Begriff "isoliert" bedeutet im Rahmen der vorliegenden Anmeldung, dass die betreffende Substanz sich nicht im Kontext ihres natürlichen Vorkommens, also innerhalb des menschlichen Körpers, befindet.

Die erfindungsgemäße Aminosäuresequenz kann die vollständige ASPP2κ Isoformen allein oder bspw. als Teil eines Fusionsproteins beinhalten. Es ist jedoch bevorzugt, wenn sie insbesondere nur die für ASPP2k spezifische Sequenz aufweist.

Eine solche Aminosäuresequenz kann im Rahmen eines Verfahrens zur Diagnose von Krebs zusammen mit einer biologischen Probe isoliert und als Marker eingesetzt werden.

Darüber hinaus kann eine erfindungsgemäße Aminosäuresequenz auch der Generierung oder Identifizierung spezifischer Bindereagenzien dienen, die bei einem solchen Verfahren oder im Rahmen der Kuration eingesetzt werden.

Hierzu kann die Aminosäuresequenz durch bekannte Verfahren, also durch Expression eines Vektors in einem Mikroorganismus, durch in vitro Translation oder durch chemische Synthese erzeugt werden. In diesem Zusammenhang ist unter einem Mikroorganismus insbesondere ein Bakterium oder eine eukaryotische Zelle, beispielsweise eine isolierte, immortalisierte Säugerzelle, eine Hefezelle oder ein filamentöser Pilz zu verstehen.

Wie erwähnt, kann eine so erzeugte Aminosäuresequenz, die die für ASPP2κ Isoformen spezifische Sequenz umfasst, zur Erzeugung bzw. Identifizierung spezifischer Bindereagenzien oder Nachweismoleküle genutzt werden. Hierzu wird bspw. ein nicht-menschlicher Organismus, vorzugsweise ein Säugetier, gegen die Aminosäuresequenz immunisiert. Anschließend können mittels bekannter Techniken Antikörper, die die Aminosäuresequenz spezifisch binden, bzw. solche Antikörper exprimierende Zellen gewonnen werden. Alternativ kann die für ASPP2κ Isoform spezifische Sequenz auch zum Screening für andere Bindereagenzien wie z.B. andere Proteine oder Small Molecules verwendet werden.

Unter Small Molecules ist in diesem Zusammenhang eine heterogene Gruppe kleiner organischer Moleküle mit einer Größe meist unterhalb 800 Dalton (Da) zu verstehen, bspw. Sekundärmetabolite oder synthetisch erzeugte Abwandlungen hiervon.

Daneben betrifft vorliegende Erfindung auch eine zugeordnete isolierte Nukleinsäuresequenz, die aus der Gruppe umfassend a) eine Nukleinsäuresequenz, die eine erfindungsgemäße Aminosäuresequenz mit der Seq. ID Nr. 1 oder 2 kodiert, b) eine Nukleinsäuresequenz, die eine Sequenz mit mindestens 90% Sequenzidentität zu der Sequenz mit der SEQ ID Nr. 3 aufweist, c) eine Nukleinsäuresequenz, die zumindest teilweise, also für zumindest 3 der Aminosäuren der Aminosäuresequenz mit der SEQ ID Nr. 2 codiert, sowie für einen N-terminal daran jeweils anschließenden Bereich von mindestens 2 Aminosäuren, und d) eine zu a), b) oder c) komplementäre bzw. reverskomplementäre Nukleinsäuresequenz ausgewählt ist.

Bei der Nukleinsäuresequenz gemäß Seq. ID Nr. 3 handelt es sich um die schon erwähnte Sequenz der ASPP2 Transkriptvariante κ, die durch den im Zuge des fehlerhaften Splicevorganges entstehenden Übergang zwischen ASPP2 Exon 16 und ASPP2 Exon 17 zustande kommt.

In diesem Zusammenhang wird im Rahmen der vorliegenden Anmeldung unter dem Begriff "komplementär" bzw. "reverskomplementär" die Fähigkeit einer Nukleinsäuresequenz verstanden; sich unter durchgängiger Bildung kanonischer Nukleotidbasenpaare wie AU, AT und GC an eine andere Nukleinsäuresequenz physikalisch bzw. physikochemisch zu binden. Eine solche Bindung erfolgt beispielsweise zwischen den Einzelsträngen einer doppelsträngigen DNA sowie, während der Transkription, zwischen einem als Vorlage dienenden DNA-Strang und einem neu gebildeten RNA-Strang, sowie während der Translation zwischen einem bestimmten Bereich einer Messenger-RNA und einem bestimmten Bereich einer Transfer-RNA.

Eine solche Nukleinsäuresequenz kann, ebenso wie eine obengenannte Aminosäuresequenz, im Rahmen eines Verfahrens zur Diagnose von Krebs zusammen mit einer biologischen Probe isoliert und als Marker eingesetzt werden.

Unter "Transkription" wird im Rahmen der vorliegenden Anmeldung die Bildung einer Ribonukleinsäuresequenz (RNA) auf Basis einer Desoxyribonukleinsäuresequenz (DNA) verstanden, während unter "reverser Transkription" die Bildung einer Desoxyribonukleinsäuresequenz auf Basis einer Ribonukleinsäuresequenz verstanden wird.

Unter Nukleinsäure-Sonden und -Primern sind in diesem Zusammenhang insbesondere einzelsträngige DNA oder RNA Sequenzen kurzer bis mittlerer Länge (ca. 10 b bis zu mehreren 100 b) zu verstehen, die unter stringenten Bedingungen an komplementäre DNA oder RNA Sequenzen hybridisieren können. Entsprechende Hybridisierungsbedingungen sind dem Fachmann, bspw. im Zusammenhang mit Techniken wie Polymerase-Kettenreaktion, Southern- oder Northern-Blot umfangreich bekannt.

Da verschiedene Organismen, beispielsweise Modellorganismen oder Organismen zur biotechnologischen Herstellung von Aminosäuresequenzen bzw. Proteinen oft vom Menschen abweichende Kodonpräferenzen zeigen, kann es im Zuge der biotechnologischen Erzeugung einer Aminosäuresequenz notwendig sein, eine Nukleinsäuresequenz durch Ersetzen bestimmter Kodons durch andere, für die gleiche Aminosäure kodierende Kodons zu modifizieren. Hierdurch kann häufig eine verbesserte Expressionsleistung bzw. eine Senkung der Fehlerhäufigkeit bei der Expression erreicht werden.

Die vorliegende Erfindung offenbart ferner einen Vektor, der eine erfindungsgemäße Nukleinsäuresequenz aufweist.

Ein "Vektor" bezeichnet in diesem Zusammenhang einen genetischen oder organismischen Kontext, in den eine Nukleinsäuresequenz strukturell und/oder funktionell eingebunden ist. Beispielsweise kann unter einem Vektor ein natürlich vorkommendes oder synthetisch erzeugtes Plasmid, ein natürlich vorkommendes oder synthetisch erzeugtes Chromosom oder eine andere Nukleinsäuresequenz verstanden werden, die, neben der Möglichkeit, auf synthetischem oder natürlichem Wege eine weitere Nukleinsäuresequenz hierin zu inserieren, für die Replikation und/oder Expression dieser zusätzlichen Nukleinsäuresequenz erforderliche Sequenzen bzw. Gene aufweist. Solche Plasmide und Chromosomen sind dem Fachmann umfangreich bekannt.

Ferner kann unter einem Vektor auch ein Organismus, vorzugsweise ein Virus, verstanden werden, der zur Infiltration eukaryotischen Gewebes fähig ist und über welchen eine Nukleinsäuresequenz in eukaryotische Zellen verbracht werden kann. Solche Vektoren sind dem Fachmann aus dem Stand der Technik umfangreich bekannt.

Ein Vektor kann im Rahmen der vorliegenden Erfindung eine ASPP2κ spezifische Sequenzzusammensetzung enthalten, die beispielsweise in den Kontext eines vollständigen, für ASPP2κ Isoformen kodierenden offenen Leserasters (ORF) eingebunden ist. Die ASPP2κ spezifische Sequenzzusammensetzung kann darüber hinaus in einen für ein Fusionsprotein kodierenden ORF eingebunden sein. Weiterhin ist es auch möglich, mit der ASPP2κ spezifischen Sequenzzusammensetzung in bekannter Weise RNA-Interferenz Konstrukte zu erzeugen. Dies geschieht beispielsweise durch Generierung einer palindromischen Sequenz oder eines Hairpin-Loops, mithilfe dessen sich eine doppelsträngige RNA mit einer ASPP2κ spezifischen Sequenzzusammensetzung erzeugen lässt.

Weiterhin betrifft die vorliegende Erfindung ein Bindereagenz, das spezifisch an eine erfindungsgemäße Nukleinsäuresequenz oder an eine erfindungsgemäße Aminosäuresequenz, wie in den Ansprüchen definiert, bindet. Hierbei ist insbesondere bevorzugt, wenn das Bindereagenz aus der Gruppe ausgewählt ist, die a) Nukleinsäure-Sonden und -Primer, b) Antikörper einschließlich Antikörperderivate, und c) Small Molecules umfasst.

In diesem Zusammenhang ist unter "Antikörperderivaten" eine von einem Antikörper abgeleitete Struktur, beispielsweise ein Antikörper-Fragment oder ein chimärer Antikörper zu verstehen. Eine Sonderform des chimeren Antikörpers ist der beispielsweise in murinen Zellen exprimierte humanisierte Antikörper, in dem sämtliche nicht direkt an der spezifischen Bindung an das Antigen beteiligten Sequenzanteile durch humane Antikörpersequenzen ausgetauscht sind. So kann eine Immunantwort des menschlichen Körpers gegen die unspezifischen Antikörperanteile verhindert bzw. modifiziert werden.

Ein solches, für ASPP2 Transkriptvariante κ oder ASPP2κ Isoformen spezifisches Bindereagenz kann im Rahmen der Diagnostik eingesetzt werden, um ASPP2 Transkriptvariante κ oder der ASPP2κ Isoformen in biologischen Proben des menschlichen oder tierischen Körpers zu binden und somit deren direkten oder indirekten Nachweis zu ermöglichen.

Hierzu kann das Bindereagenz für die direkte Detektion eingerichtet sein, indem es bspw. ein Fluorophor, einen Katalysator, ein radioaktives Isotop, ein magnetisches Partikel oder andere detektierbare Materialien oder Eigenschaften aufweist. Alternativ können solche detektierbaren Materialien oder Eigenschaften auch an sekundären Bindereagenzien, bspw. Sekundärantikörpern, vorgesehen sein, die spezifisch das erste Bindereagenz binden. Solche Detektions-Materialien, sekundäre Bindereagenzien und Verfahren sind dem Fachmann umfangreich aus dem Stand der Technik bekannt.

Entsprechend betrifft die vorliegende Erfindung auch ein diagnostisches Kit, das ein oder mehrere Reagenzien aus der Gruppe umfassend a) eine erfindungsgemäße Aminosäuresequenz, b) eine erfindungsgemäße Nukleinsäuresequenz, c) einen erfindungsgemäßen Vektor und d) ein erfindungsgemäßes Bindereagenz aufweist. Darüber hinaus weist das diagnostische Kit gemäß einer bevorzugten Ausführungsform einen Mikroarray auf.

Ein solches diagnostisches Kit kann für das oben beschriebene Verfahren eingesetzt werden und ermöglicht so die Detektion eines ASPP2κ Markers zum Zweck der qualitativen und ggf. quantitativen Analyse. Hierbei kann insbesondere im Fall eines Mikroarrays eine Vielzahl bekannter Genprodukte von Onkogenen bzw. Protoonkogenen sowie andere Marker (s.o.) parallel quantitativ bzw. semiquantitativ gemessen werden.

Eine solche Analyse ermöglicht also eine noch differenziertere Aussage über den voraussichtlichen Verlauf einer individuellen Krebserkrankung bzw. über deren Therapierbarkeit.

Die vorliegende Erfindung offenbart auch eine pharmazeutische Zusammensetzung, die einen oder mehrere Wirkstoffe aus der Gruppe umfassend a) eine erfindungsgemäße Aminosäuresequenz, b) eine erfindungsgemäße Nukleinsäuresequenz, c) eine Wildtyp-Nukleinsäuresequenz, d) einen erfindungsgemäßen Vektor und e) ein erfindungsgemäßes Bindereagenz aufweist. Die pharmazeutische Zusammensetzung kann ferner synthetische sog. small Molecules mit Mimikry der verloren gegangenen P53 Bindungsstellen umfassen, sowie zelluläre und antikörperbasierte Immuntherapeutika. Dabei wird unter "small molecules" vorliegend jede niedermolekulare Verbindung bzw. Wirkstoff verstanden, deren Molekülmasse etwa 800 g·mol⁻¹ nicht übersteigt und die/der aufgrund der geringen Größe in der Lage ist, in Zellen einzudringen und dort ihre Wirkung zu entfalten.

Eine pharmazeutische Komposition kann dabei zusätzlich zu einem oder mehreren Wirkstoffen auch eine Reihe Arzneistoffträgern und/oder Hilfsstoffen aufweisen, die beispielsweise der längeren Lagerfähigkeit oder der besseren Applizierbarkeit dienen. Solche Arzneistoffträger und Hilfsstoffe sind umfangreich aus dem Stand der Technik bekannt; vgl. Row et al. (2006), Handbook of Pharmaceutical Excipients, 5th Edition, Pharmaceutical Press; oder Bauer et al. (1999), Lehrbuch der Pharmazeutischen Technologie, 6. Auflage, Wissenschaftliche Verlagsgesellschaft Stuttgart mbH.

Eine solche pharmazeutische Komposition kann, wie oben ausgeführt entweder zur Vorbeugung oder Kuration von Krebserkrankungen verwendet werden. Hierbei ist es denkbar, eine konventionelle Behandlung mittels chirurgischer-, Chemo- oder Strahlentherapie durch Applikation einer erfindungsgemäßen pharmazeutischen Komposition zu unterstützen oder gar zu ersetzen. Daneben kann eine erfindungsgemäße pharmazeutische Komposition auch nach einer konventionellen Behandlung zur Prävention gegen ein Wiederauftreten der Krebserkrankung bspw. durch eine Minimale Resterkrankung eingesetzt werden.

Insbesondere kann eine erfindungsgemäße pharmazeutische Komposition dazu eingesetzt werden, ASPP2κ positive Krebszellen, also Zellen, bei denen die Apoptoseinduktion über ASPP2 und p53 gestört ist, für apoptoseinduzierende Stimuli zu resensibilisieren. In der Folge können diese Zellen entweder durch zellinterne Stimuli, das Immunsystem oder durch Tumortherapeutika wie u.a. Chemotherapie und Strahlentherapie zur Apoptose angeregt werden. Insbesondere durch die Verwendung von small molecules, die die p53-Bindestelle mimikrieren, oder durch Einsatz und Verwendung/Supplemetierung von/mit der Wildtypsequenz von ASPP2, können Patienten, bei denen die erfindungsgemäße ASPP2κ Isoform vorliegt, behandelt werden.

Die vorliegende Erfindung offenbart auch ein Verfahren zur Behandlung eines Patienten, wobei das Verfahren folgende Schritte aufweist: a) Durchführung eines erfindungsgemäßen Verfahrens zur in vitro Diagnose von Krebs, b) Applikation einer erfindungsgemäßen pharmazeutischen Komposition und c) ggf. Wiederholung der Schritte a) und/oder b).

Der Vorteil eines solchen Verfahrens liegt darin, dass einer der molekularen Ursachen der Krebserkrankung, nämlich der ASPP2-Insuffizienz und der damit verringerten Apoptose-Induktion gezielt entgegengewirkt werden kann. Das erfindungsgemäße Verfahren führt somit zu einer Verringerung der Therapierefrakterität bei ASPP2κ-positiven Krebserkrankungen.

Die Erfindung offenbart ferner ein Verfahren zur zellulären Immuntherapie zur Behandlung von Krebs. Erfindungsgemäß wird bei der zellulären Immuntherapie ausgenutzt, dass zytotoxische T-oder NK-(Natural Killer) Zellen aktiviert werden, die sich gegen (Krebs-)Zellen richten bzw. gezielt ausgerichtet werden (durch Antikörper oder antigenpräsentierende dendritische Zellen bzw. Hybride), die den erfindungsgemäßen Marker tragen, also die spezifische, 9 Aminosäuren lange Sequenz bzw. die N-terminal gelegene Fusionsstelle oder Teile hiervon (auch in Komplexbildung mit anderen Peptiden/Proteinen wie z.B. MHC Komplexe), die in den erfindungsgemäßen ASPP2 Transkriptvarianten enthalten sind. Dadurch können solche "defekten", die erfindungsgemäßen Transkriptvarianten exprimierenden Zellen, also Krebszellen, gezielt angegriffen und ausgeschaltet werden. So kann bspw. der Marker, der die erfindungsgemäße Sequenz trägt, als Antigen eingesetzt werden, um die zytotoxischen T-Zellen zu aktivieren.

Weitere Vorteile ergeben sich aus der beigefügten Beschreibung sowie den Figuren und Tabellen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Der Genlokus von ASPP2, die bekannten und die im Hause der Anmelderin neu gefundenen Genprodukte, der zelluläre Wirkmechanismus von ASPP2, sowie die Ergebnisse der im Hause der Anmelderin erzielten experimentellen Nachweise der ASPP2 Transkriptvariante k und der ASPP2 κ Isoformen in Krebszellen verschiedener Gewebe sind in den Figuren dargestellt, in denen:
Fig. 1 eine schematische Darstellung des C-terminalen Endes von ASPP2 zeigt;
Fig. 2 eine schematische Darstellung des C-terminalen Endes von ASPP2κ zeigt;
Fig. 3 eine schematische Darstellung der ASPP2 Transkriptvarianten 1, 2 zeigt;
Fig. 4 eine schematische Darstellung der ASPP2 Transkriptvariante κ zeigt;
Fig. 5 die ASPP2 RNA eines Patienten mit akuter Leukämie und positivem Nachweis von ASPP2κ zeigt; (A) Senseleserichtung; (B) Antisense-Sequenz; und
Fig. 6 eine Übersicht über den Frameshift der ASPP2 Transkriptvariante κ im Vergleich zum Wildtyp (A) und Western-Blots mit dem Nachweis der ASPP2 Transkriptvariante κ in Patienten mit akuter Leukämie (B) zeigt.

### Ausführungsbeispiele

### Beispiel 1: ASPP2 Transkriptvarianten

Figur 1 zeigt in schematischer, nicht maßstabsgetreuer Darstellung einen C-terminalen Bereich der für die ASPP2 Isoformen 1 und 2 (die sich durch unterschiedliches Splicing im N-terminalen Bereich unterscheiden) kodierenden Gensequenz. Dargestellt ist die Nucleinsäuresequenz, entlang der die C-terminalen Exons 14 bis 19 als weiße Boxen angeordnet sind. Im Bereich Exon 17-18 (Bereich des 4^{th} Ankyrin-Repeats und der SH3-Domäne) finden sich die p53-Bindungsstellen, welche die Bindung von ASPP2 Isoformen 1 und 2 an p53 Proteine und somit eine funktionale Interaktion ermöglichen. Diese funktionale Interaktion mündet in der Apoptoseinduktion.

Figur 2 zeigt, analog zu der in Fig. 1 gewählten Darstellungsform, einen C-terminalen Bereich der ASPP2κ Isoformen. Im Vergleich zu den ASPP2 Isoformen 1 und 2 wird bei ASPP2κ durch alternatives Splicing aus bislang ungeklärter Ursache Exon 17 nicht transkribiert. Damit fehlen alle jene p53 Bindungsstellen, die in Exon 17 kodieren.

### Beispiel 2: ASPP2 Translationsvarianten

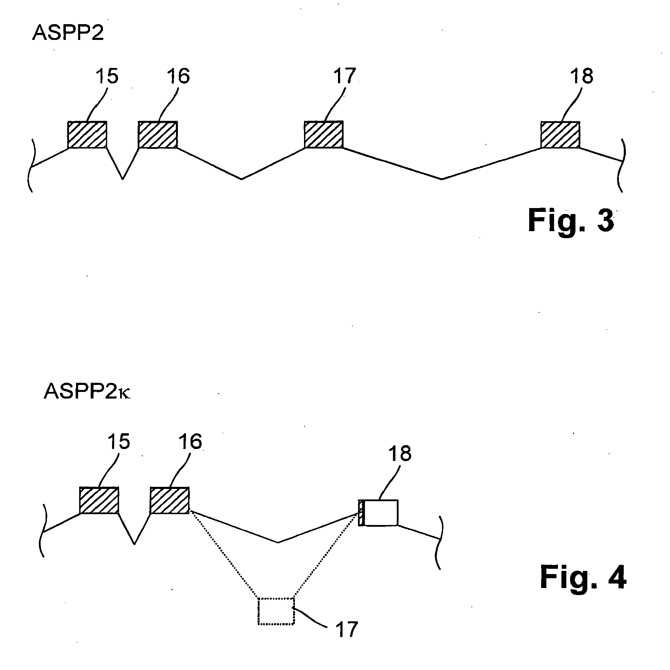

Fig. 3 zeigt in schematischer, nicht maßstabsgetreuer Darstellung einen Ausschnitt aus ASPP2 Transkriptvarianten 1 und 2. Hierbei sind die Exons (15, 16, 17, 18), also die translationsrelevanten Bereiche der Nukleinsäuresequenz, als Rechtecke dargestellt, während die im Rahmen der Transkription durch Splicing entfernten Introns durch Verbindungslinien zwischen den Exons (15, 16, 17, 18) dargestellt werden. Die Nummerierung der Exons (15, 16, 17, 18) entspricht hierbei der Exon-Nummerierung gemäß der jeweiligen Genbank Einträge (Datenbank-Nrn. NM_001031685, NM_005426). Der offene Leserahmen (ORF) mit den zu translatierenden Informationen ist als schraffierte Fläche dargestellt und füllt die hier gezeigten Exons (15, 16, 17, 18) vollständig aus.

Figur 4 zeigt, analog zu der in Fig. 3 gewählten Darstellungsform, den entsprechenden Abschnitt aus ASPP2 Transkriptvariante κ. Bei dieser Variante wurde Exon 17 während der Transkription vollständig deletiert (Fig. 1). Die sich durch die Deletion des Exons 17 ergebende Leserasterverschiebung führt zu einer für die Transkriptvariante κ neuen charakteristischen 9 Aminosäuren langen Sequenz am C-Terminus (wiedergegeben in Seq. ID Nr. 2): Der Frameshiuft führt zu einem verkürzten offenen Leserahmen mit frühzeitiger Codierung eines Stopp-Codons und damit zu einem Translationsstopp in Exon 18. Dies spiegelt sich in der in Fig. 4 schematisch dargestellten - im Vergleich zu Fig. 3 kürzeren - schraffierten Fläche im Exon 18 wieder. Resultierend werden die in Exon 18 kodierenden p53 Bindungsstellen nicht mehr translatiert.

Es zeigt sich also, dass die p53-Bindungsstellen sowohl durch die durch Leserasterverschiebung induzierte Sequenzänderung (Bsp. 2), als auch durch die Deletion von Exon 17 (Bsp. 1) vollständig deletiert und somit dysfunktional geworden sind. Eine Bindung von ASPP2 Isoform k kann somit durch diese p53-Bindungsstellen nicht mehr etabliert werden. Damit ist die durch ein Zusammenwirken von ASPP2 und p53 vermittelte Apoptoseinduktion zumindest beeinträchtigt.

### Beispiel 3: Nachweis von ASPP2κ Genprodukten in Leukämie-Zellen

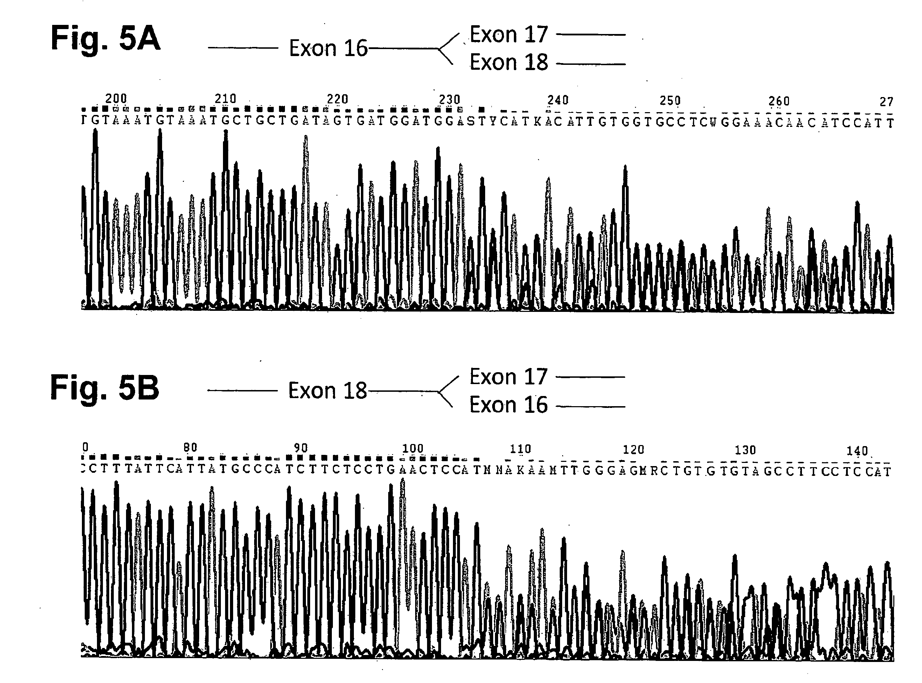

Mittels Dichtegradientenzentrifugation wurden mononukleäre Zellen einer Probe eines Patienten mit akuter Leukämie isoliert. Aus diesen wurde wiederum die mRNA nach gängigen Protokollen (Qiagen, Hilden, Deutschland) isoliert und zu cDNA revers transkribiert, die mittels PCR analysiert sowie direkt sequenziert wurde. Entsprechend sind in Fig. 5 die Ergebnisse dieser Sequenzierung gezeigt: In Fig. 5A ist die ASPP2 DNA in Senseleserichtung gezeigt, in Fig. 5B die Antisense-Sequenz.

Als Resultat des aberranten Splicings von Exon 17 bei der ASPP2 κ Transkriptvariante findet sich neben der ASPP2 Wildtypsequenz (also inklusive Exon 17) auch die kürzere Splicevariante mit direktem Anschluss von Exon 18 nach Exon 16 (also unter Auslassung von Exon 17). Die Sequenzüberlagerung markiert dabei das gleichzeitige Vorliegen zweier Sequenzen: Exon 17 und 18 in Senserichtung (Fig. 5A), bzw. Exon 17 und 16 in Antisenserichtung (Fig. 5B).

In weiteren Versuchen wurde gezeigt, dass die errechnete ASPP2κ Translationsvariante tatsächlich auch auf Proteinebene nachweisbar ist. Hierfür wurden anhand der für die ASPP2κ ermittelten 9 Aminosäure langen Sequenz (für die in einer Blast-Datenbanksuche kein Protein mit derselber Teilsequenz identifiziert werden konnte) spezifische polyklonale Kaninchen-Antikörper generiert.

In Fig. 6A ist eine Übersicht über die Exons 16 und 18 von ASPP2 gezeigt, bzw. wie der Frameshift in diesem Bereich zustande kommt. Dabei ist mit Frame 2 die ursprüngliche Wildtypsequenz gezeigt, und mit Frame 1 die Sequenz für die ASPP2κ Variante. Der Pfeil in Fig. 6A zeigt die Auslassung von Exon 17 an.

Wie Fig. 6A zu entnehmen ist, wird durch den Frameshift in der κ-Variante ein vorzeitiges Stopp-Codon generiert, so dass die C-terminalen 9 Aminosäuren der Sequenz SSGEDGHNE (Seq. ID Nr. 2 des beigefügten Sequenzprotokolls) entsprechen.

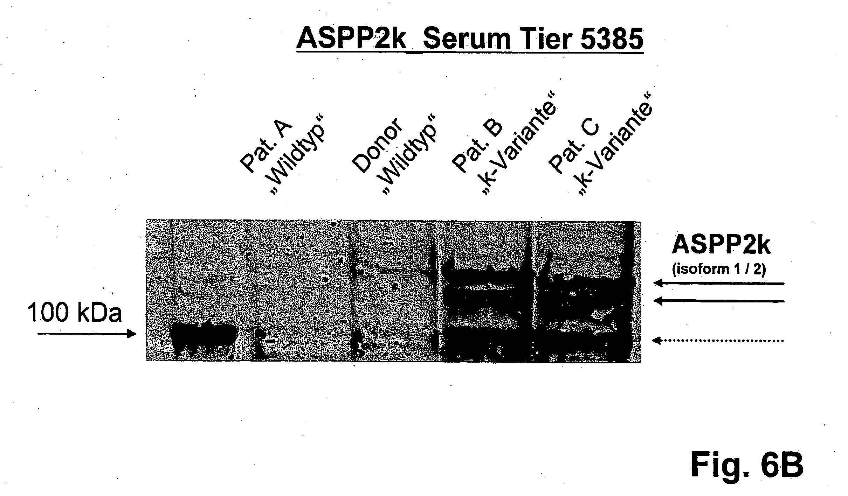

Mittels Western-Blot wurde anschließend die ASPP2κ Variante dann auch im Blut von Patienten mit akuter Leukämie nachgewiesen. Die Ergebnisse dieser Versuche sind in Fig. 6B dargestellt. Die untersuchten Proben stammten von einem gesunden Donoren ("Donor Wildtyp"), sowie von drei Patienten. Wie Fig. 6B zu entnehmen ist, wurden jeweils Mehrfachbanden bei Patient B und C nachgewiesen, die den ASPP2κ Isoformen 1 und 2 (Varianten von ASPP2 Isoformen 1 und 2) und vermutlich mindestens einer weiteren ASPP2-Varianten entsprechen, die gegenüber den Wildtyp-Isoformen ebenfalls stark verkürzt sind und die charakteristische Sequenz SSGEDGHNE tragen.

Die ASPP2 Wildtyp- bzw. kappa Varianten wurden bei dem Donoren und den Patienten A und B zuvor mittels PCR und Sequenzierung verifiziert. Bei Patient C war der ASPP2 Status zunächst nicht bekannt; das Vorliegen von ASPP2 kappa bei Patient C wurde aufgrund des Immunoblotergebnisses nahegelegt, und im Anschluss durch PCR und Sequenzierung bestätigt.

## Patentansprüche

1. Verfahren zur in vitro Bestimmung des Vorliegens oder einer Prädisposition eines Patienten für die Entwicklung von Krebs, **dadurch gekennzeichnet, dass** in einer biologischen Probe des Patienten das Vorliegen eines Markers bestimmt wird, wobei der Marker eine Splice-Variante von ASPP2 ist, und wobei der Marker ausgewählt ist aus
a) einer Aminosäuresequenz, die die Aminosäuresequenz SEQ ID Nr. 2 aus dem beigefügten Sequenzprotokoll umfasst, oder
b) oder einer Nukleinsäure, die eine Nukleinsäure umfasst, die für die Aminosäuresequenz mit der SEQ ID Nr. 2 codiert.

2. Verfahren nach Anspruch 1, **dadurch gekennzeichnet, dass** der Marker eine ASPP2 Isoform ist, die im Vergleich zu den natürlich vorkommenden ASPP2 Isoformen im c-terminalen Bereich verkürzt ist und die die Aminosäuresequenz mit der SEQ ID Nr. 2 enthält.

3. Verfahren nach einem der vorherigen Ansprüche, **dadurch gekennzeichnet, dass** der Marker ausgewählt ist aus
a) der Aminosäuresequenz mit der SEQ ID Nr. 2, oder
b) einer für die Aminosäuresequenz mit der SEQ ID Nr. 2 codierenden Nukleinsäure.

4. Verfahren nach einem der Ansprüche 2 oder 3 , **dadurch gekennzeichnet, dass** der Marker ausgewählt ist aus
einem Abschnitt der für die ASPP2 Isoform codierenden mRNA, welcher in der SEQ ID Nr. 3 die Basen Nr. 3422 bis 3448 umfasst.

5. Verfahren nach einem der vorherigen Ansprüche, **dadurch gekennzeichnet, dass** der Krebs ausgewählt ist aus hämatologischen Neoplasien, soliden Tumoren sowie Präkanzerosen, und insbesondere aus der akuten oder chronischen myeloischen oder akuten lymphatischen Leukämie.

6. Aminosäuresequenz, umfassend die mit der SEQ ID Nr. 2 wieder gegebene Aminosäuresequenz zur Verwendung als Marker zur Diagnose von Krebs.

7. Nukleinsäuresequenz, ausgewählt aus einer der folgenden:
a) Nukleinsäuresequenz codierend für eine Aminosäuresequenz mit der SEQ ID Nr. 1 oder 2,
b) Nukleinsäuresequenz, die eine zu a) komplementäre bzw. reverskomplementäre Nukleinsäuresequenz aufweist.

8. Bindereagenz, das spezifisch an eine Aminosäuresequenz mit der SEQ ID Nr. 2 bindet, oder das an eine Nukleinsäuresequenz bindet, die für zumindest 3 der Aminosäuren der Aminosäuresequenz mit der SEQ ID Nr. 2 sowie für einen N-terminal sich anschließenden Bereich von mindestens 2 Aminosäuren aus der Sequenz mit der SEQ ID Nr. 1 codiert, und das ausgewählt ist aus der Gruppe umfassend
a) Nukleinsäure-Sonden und -Primer;
b) Antikörper, einschließlich Antikörperderivate und -fragmente, insbesondere ein Antikörper, der spezifisch an die Sequenzen mit der SEQ ID Nr. 2 bindet.

9. Verfahren nach einem der Ansprüche 1 bis 5, **dadurch gekennzeichnet, dass** die für den Marker codierende mRNA oder cDNA nachgewiesen wird.

10. Verfahren nach einem der Ansprüche 1 bis 5, **dadurch gekennzeichnet, dass** der Marker mittels eines Bindereagenz nach Anspruch 8 bestimmt wird.

11. Verfahren nach Anspruch 10, mit den Schritten:
a) Inkontaktbringen einer biologischen Probe eines Patienten mit mindestens einem Bindereagenz gemäß Anspruch 8;
b) Detektion des Vorliegens von zumindest einem in der biologischen Probe enthaltenen Marker, an welchen das Bindereagenz bindet; und
c) Quantifizierung des mindestens einen Markers aus Schritt b).

12. Diagnostisches Kit für die in vitro Bestimmung des Vorliegens oder einer Prädisposition eines Patienten für die Entwicklung von Krebs, das ein oder mehrere Reagenzien aus der Gruppe umfassend
a) eine Aminosäuresequenz gemäß Anspruch 6;
b) eine Nukleinsäuresequenz gemäß Anspruch 7;
c) ein Bindereagenz gemäß Anspruch 8
aufweist.

13. Diagnostisches Kit gemäß Anspruch 12, **dadurch gekennzeichnet, dass** es ein Mikroarray aufweist.

## Claims

1. A method for the in vitro determination of the presence of or of a predisposition of a patient to the development of cancer, **characterized in that**, in a biological sample from the patient, the presence of a marker is determined, wherein the marker is a splice-variant of ASPP2, and wherein the marker is selected from
a) an amino acid sequence comprising the amino acid sequence SEQ ID No. 2 from the attached sequence listing, or
b) a nucleic acid comprising a nucleic acid encoding the amino acid sequence having SEQ ID No. 2.

2. The method as claimed in claim 1, **characterized in that** the marker is an ASPP2 isoform which, compared to the naturally occurring ASPP2 isoforms, is shortened in the C-terminal region and which contains the amino acid sequence having SEQ ID No. 2.

3. The method as claimed in one of the preceding claims, **characterized in that** the marker is selected from
a) the amino acid sequence having SEQ ID No. 2, or
b) a nucleic acid encoding the amino acid sequence having SEQ ID No. 2.

4. The method as claimed in one of claims 2 or 3, **characterized in that** the marker is selected from
a segment of the mRNA which encodes the ASPP2 isoform and which comprises bases No. 3422 to 3448 of SEQ ID No. 3.

5. The method as claimed in one of the preceding claims, **characterized in that** the cancer is selected from hematologic neoplasias, solid tumors and precancerous conditions, and in particular from acute or chronic myeloid or acute lymphoblastic leukemia.

6. An amino acid sequence comprising the amino acid sequence shown in SEQ ID No. 2 for use as a marker for the diagnosis of cancer.

7. A nucleic acid sequence, which is selected from one of the following:
a) a nucleic acid sequence coding for a amino acid sequence having SEQ ID No. 1 or 2,
b) a nucleic acid sequence which comprises a nucleic acid sequence which is complementary or reverse complementary to a).

8. A binding reagent which binds specifically to an amino acid sequence having SEQ ID No. 2, or which binds to a nucleic acid sequence which encodes at least 3 of the amino acids of amino acid sequence having SE ID No. 2 and for a region of at least 2 amino acids which is joined N-terminally thereto from the sequence having SEQ ID No. 1, and which is selected from the group comprising
a) nucleic acid probes and primers;
b) antibodies, including antibody derivatives and fragments, in particular an antibody which binds specifically to the sequences having the SEQ ID No. 2.

9. The method as claimed in any of claims 1 to 5, **characterized in that** the mRNA or cDNA encoding the marker is detected.

10. The method as claimed in any of claims 1 to 5, wherein the marker is determined using a binding reagent as claimed in claim 8.

11. The method as claimed in claim 10, comprising the steps of:
a) contacting a biological sample from a patient with at least one binding reagent as claimed in claim 8;
b) detecting the presence of at least one marker which is contained in the biological sample and to which the binding reagent binds; and
c) quantifying the at least one marker from step b).

12. A diagnostic kit for the in vitro determination of the presence or of a predisposition of a patient to the development of cancer, **characterized in that**, which comprises one or more reagents from the group comprising
a) an amino acid sequence as claimed in claim 6;
b) a nucleic acid sequence as claimed in claim 7;
c) a binding reagent as claimed in claim 8.

13. The diagnostic kit as claimed in claim 12, **characterized in that** it comprises a microarray.

## Revendications

1. Procédé de détermination in vitro de la présence ou d'une prédisposition d'un patient au développement d'un cancer, **caractérisé en ce qu'**on détermine dans un échantillon biologique du patient la présence d'un marqueur, le marqueur étant un variant d'épissage de l'ASPP2, et le marqueur étant choisi parmi
a) une séquence d'acides aminés qui comprend la séquence d'acides aminés SEQ ID NO:2 de la liste des séquences jointe, ou
b) un acide nucléique qui comprend un acide nucléique qui code pour la séquence d'acides aminés SEQ ID NO:2.

2. Procédé selon la revendication 1, **caractérisé en ce que** le marqueur est une isoforme de l'ASPP2, qui par comparaison avec les isoformes naturelles de l'ASPP2 est raccourcie dans le domaine c-terminal, et qui contient la séquence d'acides aminés SEQ ID NO:2.

3. Procédé selon l'une des revendications précédentes, **caractérisé en ce que** le marqueur est choisi parmi
a) la séquence d'acides aminés SEQ ID NO:2, ou
b) un acide nucléique codant pour la séquence d'acides aminés SEQ ID NO:2.

4. Procédé selon l'une des revendications 2 ou 3, **caractérisé en ce que** le marqueur est choisi parmi un segment de l'ARNm codant pour l'isoforme de l'ASPP2, qui dans SEQ ID NO:3 comprend les bases N° 3422 à 3448.

5. Procédé selon l'une des revendications précédentes, **caractérisé en ce que** le cancer est choisi parmi les néoplasies hématologiques, les tumeurs solides ainsi que les pré-cancéroses, et en particulier parmi la leucémie myéloïde aiguë ou chronique, ou la leucémie lymphatique aiguë.

6. Séquence d'acides aminés comprenant la séquence d'acides aminés représentée par SEQ ID NO:2, pour utilisation en tant que marqueur pour le diagnostic du cancer.

7. Séquence d'acide nucléique, choisie parmi l'une des suivantes :
a) une séquence d'acide nucléique codant pour une séquence d'acides aminés SEQ ID NO:1 ou 2,
b) une séquence d'acide nucléique qui comprend une séquence d'acide nucléique ou complémentaire ou inverse de a).

8. Réactif de liaison, qui se lie spécifiquement à une séquence d'acides aminés SEQ ID NO:2, ou qui se lie à une séquence d'acide nucléique qui code pour au moins trois des acides aminés de la séquence d'acides aminés SEQ ID NO:2, ainsi que pour un domaine, contigu au site N-terminal, d'au moins deux acides aminés de la séquence SEQ ID NO:1, et qui est choisi dans le groupe comprenant
a) les sondes et amorces à acide nucléique,
b) les anticorps, y compris les dérivés et fragments d'anticorps, en particulier un anticorps qui se lie spécifiquement aux séquences SEQ ID NO:2.

9. Procédé selon l'une des revendications 1 à 5, **caractérisé en ce qu'**on détecte l'ARNm ou l'ADNc codant pour le marqueur.

10. Procédé selon l'une des revendications 1 à 5, **caractérisé en ce que** le marqueur est déterminé à l'aide d'un réactif de liaison selon la revendication 8.

11. Procédé selon la revendication 10, comportant les étapes suivantes :
a) mise en contact d'un échantillon biologique d'un patient avec au moins un réactif de liaison selon la revendication 8 ;
b) détection de la présence d'au moins un marqueur présent dans la sonde biologique, auquel se lie le réactif de liaison ; et
c) quantification du ou des marqueurs de l'étape b).

12. Trousse diagnostique pour la détermination in vitro de la présence ou d'une prédisposition d'un patient au développement d'un cancer, qui comprend un ou plusieurs réactifs du groupe comprenant
a) une séquence d'acides aminés selon la revendication 6 ;
b) une séquence d'acides aminés selon la revendication 7 ;
c) un réactif de liaison selon la revendication 8.

13. Trousse diagnostique selon la revendication 12, **caractérisée en ce qu'**elle comprend un micro-réseau.
